# EUROPÄISCHE PATENTSCHRIFT

(11) **EP 2 048 127 B1**
(45) Veröffentlichungstag und Bekanntmachung des Hinweises auf die Patenterteilung: **09.06.2010**
(21) Anmeldenummer: 07118101.0
(22) Anmeldetag: 09.10.2007
(51) Int. Cl.: C07C 43/162, A61Q 15/00

(54) **2-Alkoxymethyl-3-isoalkenyl-1-methylcyclopentene, deren Verwendung insbesondere als Riechstoffe, entsprechende Artikel sowie Herstellverfahren**
2-alkoxy methyl-3-isoalkenyl-1-methyl cyclopentenes, their application in particular as olfactory substances, corresponding articles and manufacturing method
2-akoxymethyl-3-isoalkenyl-1-methylcyclopentène, leur utilisation, en particulier comme parfums, articles correspondants ainsi que procédé de fabrication

(43) Veröffentlichungstag der Anmeldung: 15.04.2009
(73) Patentinhaber: Symrise GmbH & Co. KG, 37603 Holzminden (DE)
(72) Erfinder: Panten, Johannes, 37671, Höxter (DE); Surburg, Horst, 37603, Holzminden (DE); Obrocki, Thomas, 30982, Pattensen (DE); Dilk, Erich, 37603, Holzminden (DE)
(74) Vertreter: Eisenführ, Speiser & Partner

(56) Entgegenhaltungen:
- CH-A5- 579 015
- DATABASE CAPLUS [Online] CHEMICAL ABSTRACTS SERVICE, COLUMBUS, OHIO, US; 1980, PODLEJSKI, JERZY ET AL: "Production of odoriferous substances by cyclization of .beta.-alkyl-.epsilon.-ketoaldehydes" XP002472127 gefunden im STN Database accession no. 1982:6878 & ZESZYTY NAUKOWE - POLITECHNIKA LODZKA, SERIA: CHEMIA SPOZYWCZA , 361(35), 341-52 CODEN: ZNLSAZ; ISSN: 0528-9254, 1980,

## Beschreibung

Die vorliegende Erfindung betrifft neue Verbindungen (2-Alkoxymethyl-3-isoalkenyl-1-methylcyclopentene) einer Formel (A) 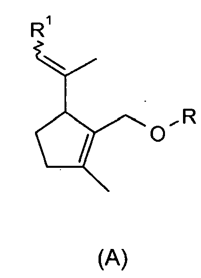 Hinsichtlich der Bedeutung der Gruppen R und R¹ siehe unten. Die vorliegende Erfindung betrifft ferner die Verwendung der neuen Verbindungen als Riech- und/oder Aromastoff und als Mittel zum Erhöhen des über einer tensidhaltigen wässrigen Lösung wahrgenommenen Geruchs anderer Riechstoffe, entsprechende Artikel umfassend eine oder mehrere erfindungsgemäße Verbindungen der Formel (A), entsprechende Riech- und Aromastoffkompositionen, entsprechende Verfahren zum Vermitteln, Modifizieren und/oder Verstärken eines Geruchs oder Geschmacks sowie entsprechende Herstellverfahren. Weitere Aspekte der vorliegenden Erfindung ergeben sich aus der nachfolgenden Beschreibung, den Beispielen sowie den beigefügten Patentansprüchen.

Trotz einer Vielzahl bereits vorhandener Riechstoffe besteht in der Parfümindustrie auch weiterhin ein genereller Bedarf an neuen Riechstoffen. Insbesondere besteht ein Bedarf an Riechstoffen, die sich durch neue originelle Duftnoten auszeichnen und solchen, die über ihre geruchlichen Eigenschaften hinaus zusätzliche positive Sekundäreigenschaften besitzen, wie z. B. eine höhere Stabilität bzw. Ausgiebigkeit unter bestimmten Anwendungsbedingungen, ein besseres Haftungsvermögen, ein sehr gutes Blooming, eine größere Diffusivität oder auch einen niedrigeren Schwellenwert.

Insbesondere besteht auch ein Bedarf an Riech- oder Aromastoffen mit komplexen Geruchs- beziehungsweise Geschmackseigenschaften, wie z.B. einer Kombination aus fruchtigen, blumigen, süßen und osmanthusartigen Aspekten, die in der Lage sind, in Riechstoff-, insbesondere Parfümkompositionen, oder Aromastoffkompositionen eine Kombination aus fruchtigen (insbesondere Aprikose), blumigen (insbesondere Flieder), süßen und osmanthusartigen Geruchs- beziehungsweise Geschmacksnoten zu erzeugen. Der vorliegenden Erfindung liegt somit die Aufgabe zugrunde, derartige Riech-und Aromastoffe anzugeben.

Erfindungsgemäß wird diese Aufgabe gelöst durch neue Verbindungen (2-Alkoxymethyl-3-isoalkenyl-1-methylcyclopentene) der Formel (A) (siehe unten) 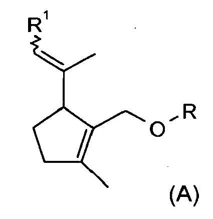 wobei unabhängig voneinander für die Gruppen R und R¹ gilt:
R ist Methyl oder Ethyl, und
R¹ ist Wasserstoff oder Methyl, wobei die geschlängelte Linie anzeigt, dass für R¹ = Methyl die zugehörige Doppelbindung (E)- oder (Z)-konfiguriert ist.

Die vorliegende Erfindung betrifft auch die entsprechende Verwendung von Verbindungen der Formel (A) als Riech- und/oder Aromastoff, entsprechende Artikel umfassend eine oder mehrere Verbindungen der Formel (A), insbesondere Riech- oder Aromastoffkompositionen, (parfümierte/aromatisierte) Artikel, die eine sensorisch wirksame Menge einer oder mehrerer 2-Alkoxymethyl-3-isoalkenyl-1-methylcyclopentene enthalten, entsprechende Verfahren zum Vermitteln, Modifizieren und/oder Verstärken eines Geruchs oder Geschmacks, Herstellungsverfahren für die erfindungsgemäßen Verbindungen der Formel (A) sowie Herstellungsverfahren für erfindungsgemäße Artikel (insbesondere Riech- oder Aromastoffkompositionen).

Der Erfindung liegt unter anderem die überraschende Erkenntnis zugrunde, dass sich die erfindungsgemäßen 2-Alkoxymethyl-3-isoalkenyl-1-methylcyclopentene der Formel (A) als Riech- und Aromastoffe eignen.

Die Formel (A) umfasst die folgenden Verbindungen:

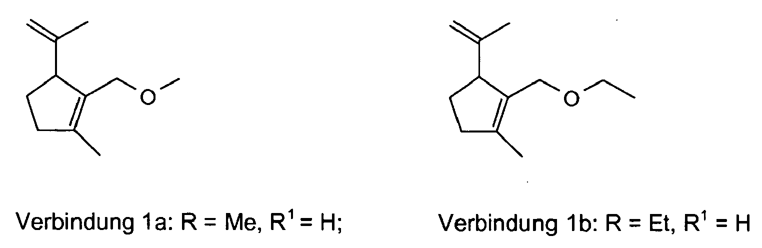

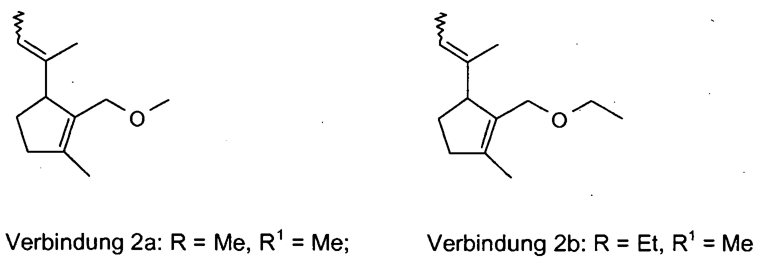

Die geschlängelte Linie im Falle der Verbindungen 2a und 2b zeigt dabei an, dass die zugehörige Doppelbindung entweder (E)- oder (Z)-konfiguriert ist. Nachfolgend werden die (E)-konfigurierten Isomere der Verbindung 2a bzw. 2b als 2aE bzw. 2bE bezeichnet, entsprechend werden die (Z)-konfigurierten Isomere als 2aZ bzw. 2bZ bezeichnet.

Die Verbindungen 1a, 1 b, 2aE, 2aZ, 2bE und 2bZ sind neu.

In eigenen Untersuchungen hat sich insbesondere herausgestellt, dass sich die erfindungsgemäßen Verbindungen der Formel (A) hervorragend zum Vermitteln, Modifizieren und/oder Verstärken der Geruchsnoten fruchtig (insbesondere Aprikose), blumig (insbesondere Flieder), süß und osmanthusartig (osmanthus = komplexer, blumig-fruchtiger Duft von Osmanthus fragrans) eignen. Individuelle Geruchsbeschreibungen sind in der nachfolgenden Tabelle angegeben:

| Verbindung/Isomerenmischung | Geruchsbeschreibung |
|---|---|
| 1a | Fruchtig (Aprikose), süß, osmanthus |
| 1b | Osmanthusartig, Aprikose, süß, Flieder, stark |
| 2aE/2aZ* | Stark osmanthus, fruchtig (Aprikose), süß, blumig |
| 2bE/2bZ* | Stark osmanthus, fruchtig (Aprikose), süß, blumig |

| | |
|---|---|
| *: in den (E)/(Z)-Isomerenmischungen lag das (E)/(Z)-Isomerenverhältnis bei 55 : 35. | |

Ferner wurden für die erfindungsgemäßen Verbindungen der Formel (A) bei der Verkostung die folgenden geschmacklichen Eigenschaften gefunden: fruchtig, blumig, süß und osmanthusartig. Es hat sich zudem herausgestellt, dass sich die erfindungsgemäßen Verbindungen der Formel (A) hervorragend zum Vermitteln, Modifizieren und/oder Verstärken der Geschmacksnoten fruchtig (insbesondere Aprikose), blumig (insbesondere Flieder), süß und osmanthusartig eignen.

Im Unterschied zu den erfindungsgemäßen Verbindungen der Formeln 2a bzw. 2b zeigen die Verbindungen 3a bzw. 3b überraschenderweise einen nur schwachen Geruch beziehungsweise Geschmack. 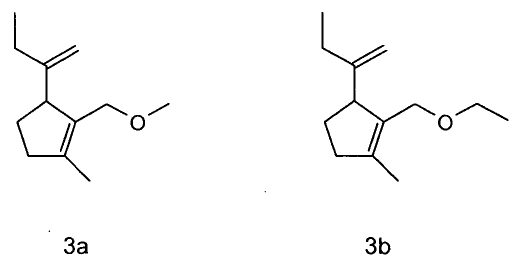 Die Verbindungen 3a bzw. 3b liegen häufig als Nebenkomponenten neben den erfindungsgemäßen Verbindungen der Formeln 2a bzw. 2b vor. Die nicht erfindungsgemäßen Verbindungen der Formeln 3a bzw. 3b lassen sich aber von den erfindungsgemäßen Verbindungen der Formeln 2a bzw. 2b durch übliche Trennverfahren, wie beispielsweise Destillation, abtrennen.

Erfindungsgemäß besonders bevorzugt ist die Verbindung der Formel 1b (Verbindung der Formel (A) mit R = Ethyl und R¹ = Wasserstoff). Die Verbindung der Formel 1b ist eine Verbindung mit einer besonders fruchtigen (Aprikose), blumigen (Flieder), süßen und osmanthusartigen Geruchs- beziehungsweise Geschmacksnote. Die Verbindung der Formel 1b hat einen besonders ausdrucksstarken fruchtigen (Aprikose), blumigen (Flieder), süßen und osmanthusartigen Geruch beziehungsweise Geschmack, was besonders überraschend ist, da (a) sie sich strukturell recht deutlich von bekannten Substanzen mit den oben beschriebenen Geruchseigenschaften unterscheidet, und weil (b) die strukturell wohl ähnlichste bekannte Verbindung der Formel 4b (siehe unten) keinen vergleichbaren Geruch besitzt.

Die nachfolgende Tabelle 1 zeigt ausgewählte Verbindungen, die wesentlich zum sensorischen Charakter des Osmanthusöles (Osmanthus fragrans) beitragen (Quelle: G. Ohloff, Riechstoffe und Geruchssinn, S. 164, Springer-Verlag, Berlin, 1990):

**Tabelle 1:**

| Struktur | Geruchseigenschaften |
|---|---|
| 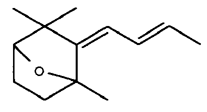 | Cassiaknospen, Tomatenblüte, exotische Früchte |
| 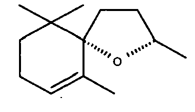 | Sensorisches Prinzip des Teearomas |
| 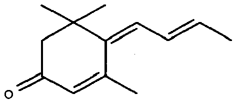 | Holzig, balsamisch, tabakartig |

Man erkennt, dass die für den sensorischen Charakter des Osmanthusöles verantwortlichen Verbindungen strukturell deutlich unterschiedlich zu den erfindungsgemäßen Verbindungen der Formel (A) und insbesondere zu der besonders bevorzugten Verbindung der Formel 1 b sind.

Strukturell zu den erfindungsgemäßen Verbindungen ähnliche Verbindungen sind die Verbindungen der Formeln 4a und 4b: 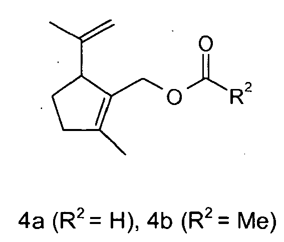 Diese Verbindungen der Formeln 4a und 4b besitzen jedoch sehr deutlich andere Geruchs- beziehungsweise Geschmackseigenschaften als die erfindungsgemäßen Verbindungen. Die sensorischen Eigenschaften der Verbindungen 4a und 4b werden wie folgt beschrieben:
4a: holzig, patchouliartig (CH 579,015),
4b: ozonig, stechend (Zeszyty Naukowe-Politechnika Lodzka; Seria: Chemia Spozywcza (1980), 361 (35), 341-52).

Insgesamt war es somit also besonders überraschend, dass jede der erfindungsgemäßen Verbindungen, insbesondere jedoch die besonders bevorzugte Verbindung der Formel 1b eine besonders starke fruchtige (aprikosenartige), blumige (Flieder), süße und osmanthusartige Geruchsbeziehungsweise Geschmacksnote aufweist.

Die besonderen olfaktorischen Eigenschaften und die hervorragenden stofflichen Eigenschaften, wie Löslichkeit in üblichen kosmetischen Lösungsmitteln, Kompatibilität mit weiteren Bestandteilen derartiger Produkte, etc., unterstreichen die besondere Eignung der erfindungsgemäßen Verbindungen der Formel (A) für die genannten Einsatzzwecke.

Entsprechende Aspekte der vorliegenden Erfindung betreffen die Verwendung einer Verbindung der Formel (A), insbesondere in einer der vorstehend als besonders bevorzugt angegebenen Ausgestaltungen, als Riech- und/oder Aromastoff.

Besonders bevorzugt ist eine erfindungsgemäße Verwendung einer Verbindung der Formel (A) als Riech- und/oder Aromastoff zum Vermitteln, Modifizieren und/oder Verstärken einer fruchtigen (insbesondere Aprikose), blumigen (insbesondere Flieder), süßen und/oder osmanthusartigen Geruchs- oder Geschmacksnote.

Gesucht werden - insbesondere für die Parfümierung von tensidhaltigen Formulierungen, wie zum Beispiel für Shampoos, Waschmittel oder Weichspüler - häufig Riechstoffkompositionen mit einer fruchtigen, blumigen, süßen und osmanthusartigen Note, wobei diese gleichzeitig ein ausgeprägtes Blooming (Geruch aus einer wässrigen Tensidlösung) aufweisen sollen. Die erfindungsgemäßen 2-Alkoxymethyl-3-isoalkenyl-1-methylcyclopentene der Formel (A) (das heißt der Formeln 1a, 1b, 2aE, 2aZ, 2bE, 2bZ) zeigen in einem direkten Performance-Test mit einer Vergleichsubstanz hervorragende Eigenschaften und eignen sich deshalb gut zum "Blooming" von Tensidlösungen (Panel-Bewertung siehe unten). Die vorliegende Erfindung betrifft somit auch die Verwendung einer Verbindung der Formel (A) zum Erhöhen des über einer tensidhaltigen wässrigen Lösung wahrgenommenen Geruchs anderer Riechstoffe. Hinsichtlich bevorzugter Ausgestaltungen der Verbindung der Formel (A) gilt dabei das weiter oben Gesagte entsprechend.

Die vorliegende Erfindung betrifft gemäß einem weiteren Aspekt auch Artikel umfassend eine oder mehrere Verbindungen der Formel (A) wie oben definiert. Sofern ein erfindungsgemäßer Artikel nur eine Verbindung der Formel (A) umfasst, also nur eine der Verbindungen 1a, 1b, 2aE, 2aZ, 2bE und 2bZ, umfasst der Artikel zumindest noch eine sonstige Substanz.

Bevorzugte erfindungsgemäße Artikel umfassen oder bestehen jedoch aus zwei oder mehr Verbindungen der Formel (A) wie weiter oben definiert.

Insbesondere im Falle der Verbindungen der Formel 2a bzw. 2b ist es vorteilhaft, Mischungen der jeweiligen (E)- und (Z)-Isomere einzusetzen. Dementsprechend besteht ein bevorzugter erfindungsgemäßer Artikel aus einer ersten und einer zweiten Verbindung der Formel (A) wie oben definiert, wobei in der ersten und der zweiten Verbindung R identisch ist, R¹ Methyl ist und die zugehörige Doppelbindung der ersten Verbindung (E)-konfiguriert und die der zweiten Verbindung (Z)-konfiguriert ist.

Besonders bevorzugte erfindungsgemäße Artikel umfassen eine Gesamtmenge an Verbindungen der Formel (A) wie oben definiert, die ausreicht, um eine, zwei, drei oder sämtliche Geruchs- oder Geschmacksnoten aus der Gruppe bestehend aus fruchtig (insbesondere Aprikose), blumig (insbesondere Flieder), süß und osmanthusartig zu vermitteln, zu modifizieren und/oder zu verstärken.

Ein besonders relevanter Aspekt der vorliegenden Erfindung betrifft Riech- und Aromastoffkompositionen, welche eine oder mehrere Verbindungen der Formel (A) umfassen. Entsprechend ist ein bevorzugter erfindungsgemäßer Artikel eine solche Riech- oder Aromastoffkomposition, umfassend eine oder mehrere Verbindungen der Formel (A) wie oben definiert sowie einen oder mehrere weitere Riech- beziehungsweise Aromastoffe. Hinsichtlich der bevorzugten Auswahl des einen oder der mehreren weiteren Riech- beziehungsweise Aromastoffe siehe weiter unten.

Bevorzugte erfindungsgemäße Artikel umfassen häufig einen Träger oder ein Substrat, der bzw. das in direktem Kontakt mit der oder den erfindungsgemäßen Verbindungen der Formel (A) bzw. der erfindungsgemäßen Riech- oder Aromastoffkomposition (als bevorzugtem Beispiel eines erfindungsgemäßen Artikels) steht.

Eine bevorzugte erfindungsgemäße Riech- oder Aromastoffkomposition umfasst eine Gesamtmenge von Verbindungen der Formel (A) wie oben definiert im Bereich von 0,001 bis 70 Gew.-%, vorzugsweise 0,05 bis 50 Gew.-% und besonders bevorzugt 0,5 bis 25 Gew.-%, bezogen auf die Gesamtmenge der Riech- beziehungsweise Aromastoffkomposition.

Hinsichtlich der in einer erfindungsgemäßen Riech- oder Aromastoffkomposition bevorzugt einzusetzenden Verbindungen der Formel (A) gilt das oben hinsichtlich der bevorzugten Verbindungen der Formel (A) Gesagte entsprechend.

Von großem Interesse für die parfümistische Kompositionsarbeit sind ferner Riechstoffe, die selbst eine ausgeprägte Diffusivität (Raumwirkung) aufweisen und/oder die Diffusivität eines Parfümöls (Riechstoffkompositionen) verbessern. Die erfindungsgemäßen Verbindungen der Formel (A), das heißt die Verbindungen der Formel 1a, 1b, 2aE, 2aZ, 2bE und 2bZ, zeigen diese beiden Wirkungen.

Die vorliegende Erfindung betrifft auch ein Verfahren zum Vermitteln, Modifizieren und/oder Verstärken eines Geruchs oder Geschmacks, wobei eine Menge (a) einer oder mehrerer Verbindungen der Formel (A) wie vorstehend definiert oder (b) einer erfindungsgemäßen Riech- oder Aromastoffkomposition mit einem Erzeugnis in Kontakt gebracht oder gemischt wird.

Es versteht sich, dass ein solches erfindungsgemäßes Verfahren insbesondere zum Vermitteln, Modifizieren und/oder Verstärken eines Geruchs oder Geschmacks mit einer, zwei, drei oder sämtlichen der Noten fruchtig (insbesondere Aprikose), blumig (insbesondere Flieder), süß und osmanthusartig eingesetzt werden wird.

Ein besonders bevorzugtes erfindungsgemäßes Verfahren zum Modifizieren und/oder Verstärken eines Geruchs oder Geschmacks mit einer, zwei, drei oder sämtlichen der Noten fruchtig (insbesondere Aprikose), blumig (insbesondere Flieder), süß und osmanthusartig, umfasst den folgenden Schritt:
- Vermischen
   einer Menge an einer oder mehreren Verbindungen der Formel (A) wie vorstehend definiert (insbesondere gemäß einer der vorstehend als besonders bevorzugt bezeichneten Ausgestaltungen)
   mit
   einem oder mehreren sonstigen Riech- oder Aromastoffen mit einer, zwei, drei oder sämtlichen der Noten fruchtig (insbesondere Aprikose), blumig (insbesondere Flieder), süß und osmanthusartig,
wobei die eingesetzte Menge der einen oder mehreren Verbindungen der Formel
(A) wie oben definiert, ausreicht, den Geruchs- beziehungsweise Geschmackseindruck der sonstigen Riech- bzw Aromastoffe, die eine, zwei, drei oder sämtliche der Noten fruchtig (insbesondere Aprikose), blumig (insbesondere Flieder), süß und osmanthusartig verursachen, sensorisch zu modifizieren und/oder zu verstärken.

Ganz entsprechend umfasst ein erfindungsgemäßes Verfahren zur Herstellung eines (erfindungsgemäßen) Artikels den folgenden Schritt:
- Vermischen einer oder mehrer Verbindungen der Formel (A) wie oben definiert (vorzugsweise gemäß einer der oben als besonders bevorzugt bezeichneten Ausgestaltungen) mit weiteren Bestandteilen, wobei eine Menge einer oder mehrer solcher Verbindungen der Formel (A) eingesetzt wird, die ausreicht, um in dem Artikel eine Geruchs- oder Geschmacksnote zu vermitteln, zu modifizieren und/oder zu verstärken.

Eine erfindungsgemäße Riech- oder Aromastoffkomposition (als Beispiel eines bevorzugten erfindungsgemäßen Artikels) wird vorzugsweise hergestellt, indem eine oder mehrere Verbindungen der Formel (A) wie oben definiert mit üblichen weiteren Bestandteilen einer Riech- oder Aromastoffkomposition vermischt werden, wobei die erfindungsgemäßen Verbindungen der Formel (A) in einer sensorisch wirksamen Menge eingesetzt werden, das heißt in einer Menge, die ausreicht, um in der Riech- oder Aromastoffkomposition eine Geruchsbeziehungsweise Geschmacksnote zu vermitteln, zu modifizieren und/oder zu verstärken. Es versteht sich, dass die eingesetzte Menge vorzugsweise ausreicht, um in der Riech- oder Aromastoffkomposition eine fruchtige (insbesondere Aprikose), blumige (insbesondere Flieder), süße und/oder osmanthusartige Geruchs- beziehungsweise Geschmacksnote (a) zu vermitteln oder (b) zu modifizieren und/oder zu verstärken (im Vergleich mit einer Mischung der sonstigen Bestandteile). Zu üblichen weiteren Bestandteilen von Riech- oder Aromastoffkompositionen finden sich weiter unten Beispiele.

Weitere besonders bevorzugte erfindungsgemäße parfümierte Produkte sind daher Luft- und Raumerfrischer, wie beispielsweise Raumsprays, Toiletten-Duftsteine oder Lufterfrischergele.

Fruchtige (insbesondere Aprikose), blumige (insbesondere Flieder), süße und osmanthusartige Geruchsnoten werden in vielfältigen Parfümkompositionen eingesetzt, z.B. in Blumenduft-Themen. Das weiter unten folgende Beispiel 1 eines "Rosen"-Duftthemas (Anwendung in Weichspüler) demonstriert in anschaulicher Weise repräsentativ den olfaktorischen Effekt der erfindungsgemäßen Verbindungen anhand von 2-Ethoxymethyl-3-isopropenyl-1-methylcyclopenten 1 b.

Die erfindungsgemäßen Verbindungen der Formel (A) können ferner eingesetzt werden, um den Geruch oder Geschmack einer Riech- oder Aromastoffkomposition zu modifizieren und/oder zu verstärken, insbesondere einer Riech- oder Aromastoffkomposition mehr Frische, Fülle, (Aus)Strahlung und/oder Abrundung zu verleihen und/oder vorhandene Geruchsbeziehungsweise Geschmacksnoten einer Riech- oder Aromastoffkomposition zu verstärken, insbesondere Geruchs- beziehungsweise Geschmacksnoten der Richtungen fruchtig (insbesondere Aprikose), blumig (insbesondere Flieder), süß und osmanthusartig.

Die vorliegende Erfindung betrifft auch Verfahren zur Herstellung einer Verbindung der Formel (A) wie oben definiert. In eigenen Untersuchungen haben sich verschiedene Verfahren als vorteilhaft herausgestellt, die jeweils die folgenden Schritte umfassen:
(i) Bereitstellen oder Herstellen einer Verbindung der Formel (C)
   sowie
(ii) Umsetzen der Verbindung der Formel (C) in einem oder mehreren Schritten, so dass eine Allylumlagerung und eine Veretherung zur Verbindung der Formel (A) resultiert.

In Anlehnung an bekannte Verfahren kann die Herstellung der erfindungsgemäßen Verbindungen der Formel (A) beispielsweise ausgehend von Dehydrolinalool (R¹ = H) bzw. 3,7-Dimethyl- -6-nonen-1-in-3-ol (R¹ = Me) in vier Schritten durchgeführt werden, vergleiche das nachfolgende Reaktionsschema: 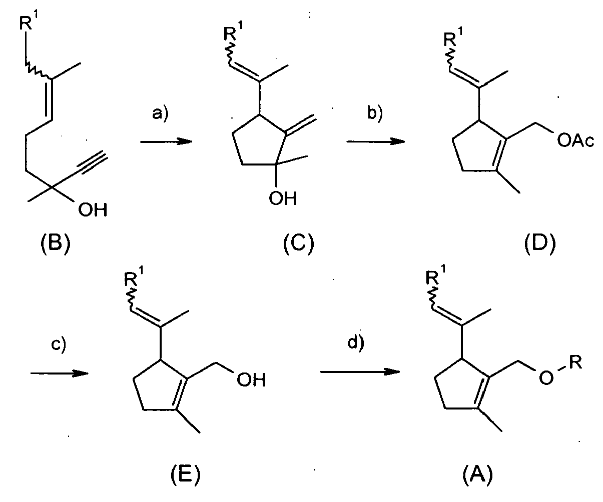 In jeder der im vorstehenden Reaktionsschema abgebildeten Formeln haben R und R¹ sowie die geschlängelte Linie eine gleichbleibende Bedeutung, wobei hinsichtlich der jeweiligen Bedeutung das weiter oben hinsichtlich der Verbindungen der Formel (A) Gesagte entsprechend gilt. Insbesondere bedeutet somit beispielsweise die geschlängelte Linie, dass die zugeordnete Doppelbindung (E)- oder (Z)-konfiguriert ist, wenn R¹ = Methyl ist.

Stufe (Schritt) a) ist eine thermische En-Reaktion. Diese thermische En-Reaktion erfolgt sehr selektiv beispielsweise durch zwölfstündiges Erhitzen der puren Substanz bei 185°C. In eigenen Untersuchungen wurde hierbei eine Ausbeute von 99 % erreicht.

Stufe (Schritt) b) ist eine Allylumlagerung. Die Allylumlagerung erfolgt regelmäßig als katalytische Allylumlagerung, wobei Wolframsäure vorzugsweise als Katalysator eingesetzt wird. Im Rahmen der Allylumlagerung wird das Produkt der Stufe a) zum Beispiel mit Essigsäureanhydrid umgesetzt, so dass sich als Produkt der Stufe b) der entsprechende Essigester bildet. In eigenen Untersuchungen wurde hier eine Ausbeute von 78% erreicht, bei einer Reaktionszeit von 2 Stunden und einer Reaktionstemperatur von 135 °C.

In Stufe (Schritt) c) wird der gebildete Ester zum Alkohol verseift. Dies geschieht vorzugsweise indem man den Ester in eine Lösung von Natriummethanolat in überschüssigem Methanol zutropft und anschließend bei Raumtemperatur rührt. Nach zweitägigem Rühren ergab sich hierbei in eigenen Untersuchungen eine Ausbeute von 90 %.

In Stufe (Schritt) d) wird der in Stufe c) erhaltene Alkohol verethert. Dies erfolgt vorzugsweise durch Einwirkung von Alkylierungsmitteln wie Diethylsulfat (DES) bzw. Dimethylsufat (DMS) unter Zusatz eines Phasentransferkatalysators, insoweit kann beispielsweise Tetrabutylammoniumbromid, TBABr, eingesetzt werden. Dabei wird der Alkohol vorzugsweise in einer Mischung aus Natronlauge (50%), Toluol und Phasentransferkatalysator vorgelegt und anschließend DES bzw. DMS bei ca. 50°C zugetropft. Zur Zerstörung des überschüssigen Alkylierungsreagenzes wird anschließend mit konzentrierter Ammoniaklösung aufgearbeitet. Nach Destillation kann der Ether so z.B. in einer Reinheit von 97 % in ca. 90%-iger Ausbeute isoliert werden.

Die vorstehend erläuterte vierstufige Synthese führt mit guten Ausbeuten zum Zielprodukt. Nachteilig ist jedoch, dass recht viele Reaktionsschritte durchgeführt werden müssen und dass in einigen Reaktionsstufen toxikologisch bedenkliche Stoffe eingestetzt werden, wie beispielsweise in Stufe d) Diethylsulfat.

Als vorteilhaft herausgestellt hat sich deshalb eine neue Herstellungsmethode, welche nicht an bereits bekannte Verfahren anknüpft. Mittels dieser neuen Herstellungsmethode gelingt die Synthese der erfindungsgemäßen Verbindungen der Formel (A) in zwei Schritten und es werden lediglich unbedenkliche Substanzen eingesetzt. Das erfindungsgemäße bevorzugte zweistufige Herstellungsverfahren ist im nachfolgenden Reaktionsschema zusammengefasst: 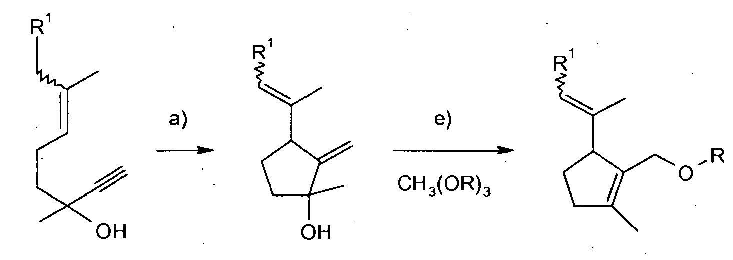 Hierin besitzen in jeder dargestellten Formel die Gruppen R und R¹ wiederum eine gleichbleibende Bedeutung. Das weiter oben Gesagte gilt jeweils entsprechend. Stufe (Schritt) a) des bevorzugten erfindungsgemäßen Herstellungsverfahrens wurde entsprechend dem weiter oben beschriebenen vierstufigen Verfahren durchgeführt. Das Reaktionsprodukt der Stufe a) wurde dann - und hierin liegt der relevante Unterschied gegenüber dem vierstufigen Verfahren - durch Erhitzen unter Rückfluss mit dem entsprechenden ortho-Ester erhalten. Eingesetzt werden können insbesondere orthoAmeisensäuretrimethylester, ortho-Ameisensäuretriethylester, ortho-Essigsäuretrimethylester sowie ortho-Essigsäuretriethylester.

Der En-Reaktion gemäß Stufe a) schließt sich bei dieser bevorzugten erfindungsgemäßen Verfahrensgestaltung in der zweiten Stufe e) eine kombinierte Allylumlagerung und Veretherung zu den gewünschten erfindungsgemäßen Verbindungen der Formel (A) in einem Schritt an. Eine ähnliche Reaktion wurde bislang nur einmal in der Literatur beschrieben, allerdings wurde dort mit einem heterogenen Katalysator und mit sogenannten Baylis-Hillman-Addukten gearbeitet, die sich von den erfindungsgemäß vorliegenden Molekülen deutlich durch das Vorhandensein einer weiteren elektronen-ziehenden Gruppe unterscheiden (Tetrahedron Lett. 2006, 47, 7619-7623).

In Stufe e) des bevorzugten erfindungsgemäßen Herstellungsverfahrens fallen neben den erfindungsgemäßen Verbindungen 2a bzw. 2b auch die schwachriechenden, nicht erfindungsgemäßen Verbindungen 3a bzw. 3b an, welche abgetrennt werden können. Auf diese Verbindungen 3a bzw. 3b wurde bereits weiter oben hingewiesen. Die Abtrennung erfolgt bei Bedarf vorzugsweise mittels üblicher Trennverfahren wie beispielsweise Destillation.

Erfindungsgemäße Artikel können als Riech- oder Aromastoffkompositionen, Riech- oder Aromastoffmischungen und Parfümöle ausgestaltet sein. Derartige Produkte können in flüssiger Form, unverdünnt oder mit einem Lösungsmittel verdünnt, für Parfümierungen eingesetzt werden. Geeignete Lösungsmittel sind z. B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw.

Für manche Anwendungen ist es vorteilhaft, Verbindungen der Formel (A) enthaltende Parfümöle (Riechstoffmischungen; Riechstoffkompositionen) oder Aromastoffkompositionen an einem Trägerstoff adsorbiert einzusetzen, der sowohl für eine feine Verteilung der Riech- beziehungsweise Aromastoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer, Cellulose-basierende Stoffe, Zucker oder Kunststoffe wie PVC, Polyvinylacetate oder Polyurethane sein. Hierbei handelt es sich um eine bevorzugte Ausgestaltung erfindungsgemäßer Artikel, welche neben einer oder mehreren Verbindungen der Formel (A) einen Träger oder ein Substrat umfassen, der bzw. das in direktem Kontakt mit der oder den Verbindungen der Formel (A) bzw. der Riech- oder Aromastoffkomposition steht. Siehe dazu oben.

Für andere Anwendungen ist es vorteilhaft, Verbindungen der Formel (A) enthaltende Parfümöle (Riechstoffmischungen; Riechstoffkompositionen) oder Aromastoffkompositionen mikroverkapselt, sprühgetrocknet, als Einschluss-Komplex oder als Extrusions-Produkt einzusetzen und in dieser Form dem zu parfümierenden oder aromatisierenden (Vor-)Produkt hinzuzufügen.

Die Eigenschaften derart modifizierter Parfümöle werden in manchen Fällen durch sogenanntes "Coaten" mit geeigneten Materialien im Hinblick auf eine gezieltere Duftfreisetzung weiter optimiert, wozu vorzugsweise wachsartige Kunststoffe wie z.B. Polyvinylalkohol verwendet werden.

Die Mikroverkapselung der erfindungsgemäßen Riech- oder Aromastoffkompositionen kann beispielsweise durch das sogenannte Koazervationsverfahren mit Hilfe von Kapselmaterialien z.B. aus polyurethanartigen Stoffen oder Weichgelatine erfolgen. Die sprühgetrockneten Riech- oder Aromastoffkompositionen können beispielsweise durch Sprühtrocknung einer die Riech- oder Aromastoffkomposition enthaltenden Emulsion, bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen derRiechoder Aromastoffkomposition und Cyclodextrinen oder Harnstoffderivaten in ein geeignetes Lösungsmittel, z.B. Wasser, hergestellt werden. Extrusions-Produkte können durch Verschmelzen der Riech- oder Aromastoffkompositionen mit einem geeigneten wachsartigen Stoff und durch Extrusion mit nachfolgender Erstarrung, ggf. in einem geeigneten Lösungsmittel, z.B. Isopropanol, erfolgen.

Verbindungen der Formel (A) enthaltende Parfümöle (Riechstoffmischungen; Riechstoffkompositionen) oder Aromastoffkompositionen können in konzentrierter Form, in Lösungen oder in sonstiger modifizierter Form verwendet werden für die Herstellung von z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendes-infektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und - lotionen, Sonnenschutzcremes- und -lotionen, After-sun-cremes und -lotionen, Handcremes und -lotionen, Fußcremes und -lotionen, Enthaarungscremes und - lotionen, After-shave-Cremes und -lotionen, Bräunungscremes und -lotionen, Haarpflegeprodukten wie z.B. Haarsprays, Haargelen, Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes oder Produkten der dekorativen Kosmetik.

Zutaten, mit denen eine oder mehrere der erfindungsgemäßen Verbindungen der Formel (A) kombiniert werden können und mit denen sie dann gemeinsam einen erfindungsgemäßen Artikel bilden, sind beispielsweise:

Konservierungsmittel, Abrasiva, Antiakne-Mittel, Mittel gegen Hautalterung, anitbakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweisshemmende Mittel, antiseptische Mittel, Antistatika, Binder, Puffer, Trägermaterialien, Chelatbildner, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Silicone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmacksstoffe, Riechstoffe, Polyole, Tenside, Elektrolyte, organische Lösungsmittel oder Silikonderivate.

Die erfindungsgemäßen Verbindungen der Formel (A) eignen sich wegen ihrer olfaktorischen Eigenschaften vorzüglich für den Einsatz in Riechstoffkompositionen (Riechstoffmischungen; Parfümölen) oder Aromastoffkompositionen. Die erfindungsgemäßen Verbindungen können dabei als Einzelstoff oder mit einer Vielzahl weiterer Riech- oder Aromastoffe kombiniert in zahlreichen Produkten verwendet werden. Besonders vorteilhaft lassen sich die Verbindungen mit anderen Riech- oder Aromastoffen in verschiedenen, unterschiedlichen Mengenverhältnissen zu neuartigen Parfüm- (als spezielle Riechstoffmischungen) oder Aromastoffkompositionen kombinieren. Solche Riech- oder Aromastoffkompositionen umfassen vorzugsweise zwei, drei, vier, fünf, sechs, sieben, acht, neun, zehn oder mehr der nachfolgend genannten Extrakte aus natürlichen Rohstoffen und/oder der nachfolgend genannten Einzel-Riech- oder Aromastoffe.

Beispiele für Riech- oder Aromastoffe, mit denen die erfindungsgemäßen Verbindungen zur Herstellung eines erfindungsgemäßen Artikels, insbesondere zur Herstellung einer erfindungsgemäßen Riech- oder Aromastoffkomposition vorteilhaft kombiniert werden, finden sich z. B. in S. Arctander, Perfume and Flavor Chemicals, Vol. I und II, Montclair, N. J., 1969, Selbstverlag oder H. Surburg, J. Panten, Common Fragrance and Flavor Materials, 5th ed., Wiley-VCH, Weinheim, 2006. Im Einzelnen seien genannt:

Extrakte aus natürlichen Rohstoffen wie Etherische Öle, Concretes, Absolues, Resine, Resinoide, Balsame, Tinkturen wie z. B.

Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limettenöl destilliert; Limettenöl gepresst; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;
Einzel-Riechstoffe aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; (E)-und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadiensäurenitril; 3,7-Dimethyl-6-octensäurenitril;
der Ester von aliphatischen Carbonsäuren wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
der acyclischen Terpenalkohole wie z. B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z. B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z. B. Menthol; Isopulegol; alpha-Terpineol; Terpineol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z. B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; 2-Methyl-4-(2,6,6-trimethyl-1-cyclohexen-1-yl)-2-butenal; Nootkaton; Dihydronootkaton; 4,6,8-Megastigmatrien-3-on; alpha-Sinensal; beta-Sinensal; acetyliertes Cedernholzöl (Methylcedrylketon);
der cyclischen Alkohole wie z.B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether; 1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 7-Cyclohexadecen-1-on; 8-Cyclohexadecen-1-on; 9-Cycloheptadecen-1-on; Cyclopentadecanon; Cyclohexadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat;2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Alkohole wie z.B.1-Cyclohexylethylcrotonat;
der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b--Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z.B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; I; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; 2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl--5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Die erfindungsgemäßen Verbindungen der Formel (A) sowie deren Mischungen können (in sensorisch wirksamer Menge) in aromatisierte oder zu aromatisierende Artikel eingearbeitet werden, insbesondere der Ernährung, der Mundpflege oder dem Genuss dienende Zubereitungen. Besonders bevorzugte der Ernährung oder dem Genuss dienende Zubereitungen sind süße und fruchtige Anwendungen sowie Getränke.

Der Ernährung oder dem Genuss dienende Zubereitungen sind z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nichtalkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Milchgetränke, Milcheis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, in Essig eingelegte Gemüse, eingekochte Gemüse), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder Emulsionen derselben (z.B. Mayonnaise, Remoulade, Dressings, Würzzubereitungen), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden. Nach Einarbeiten der erfindungsgemäß zu verwendenden Verbindungen der Formel (A) bzw. der Mischungen sind diese Zubereitungen erfindungsgemäße Zubereitungen.

Erfindungsgemäße Zubereitungen können z. B. als Halbfertigware oder als Würzmischung vorliegen.

Erfindungsgemäße Zubereitungen können insbesondere als Halbfertigware zur Herstellung weiterer der Ernährung oder dem Genuss dienenden Zubereitungen dienen, insbesondere in sprühgetrockneter Form. Erfindungsgemäße Zubereitungen können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Der Mundpflege dienende erfindungsgemäße Zubereitungen sind insbesondere Mund- und/oder Zahnpflegemittel wie Zahnpasten, Zahngele, Zahnpulver, Zahnseide, Zahnstocher, Mundwässer und Kaugummis.

Weitere übliche Wirk-, Grund-, Hilfs- und Zusatzstoffe für der Ernährung, der Mundpflege oder dem Genuss dienende erfindungsgemäße Zubereitungen können in Mengen von 5 bis 99,99 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99,99 Gew.-%, vorzugsweise 5 bis 80 Gew.-%, bezogen auf das Gesamtgewicht der Zubereitung, aufweisen.

Die erfindungsgemäßen Zubereitungen (als Beispiele erfindungsgemäßer Artikel), enthaltend eine oder mehrere der Verbindungen der Formel (A), oder deren Mischungen, werden gemäß einer bevorzugten Ausgestaltung hergestellt, in dem die Verbindung bzw. die Verbindungen der Formel (A) als Substanz, als Lösung (z.B. in Ethanol, Wasser oder 1,2-Propylenglycol) oder in Form eines Gemisches mit einem festen oder flüssigen Trägerstoff (z.B. Maltodextrin, Stärke, Silicagel), sonstigen Aromen oder Aromastoffen und gegebenfalls weiteren Hilfsmitteln und/oder Stabilisatoren (z.B. natürliche oder künstliche Polysaccharide und/oder Pflanzengummen wie modifizierte Stärken oder Gummi Arabicum) in eine der Ernährung, der Mundpflege oder dem Genuss dienende Basis-Zubereitung eingearbeitet werden. Vorteilhafterweise können als Lösung und/oder Suspension oder Emulsion vorliegende erfindungsgemäße Zubereitungen auch durch Sprühtrocknung in eine feste erfindungsgemäße Zubereitung (Halbfertigware) überführt werden.

Die erfindungsgemäßen sprühgetrockneten festen Zubereitungen (als Beispiel erfindungsgemäßer Artikel) sind als Halbfertigwaren besonders gut zur Herstellung von weiteren erfindungsgemäßen Zubereitungen geeignet. In den erfindungsgemäßen sprühgetrockneten festen Zubereitungen sind vorzugsweise 50 bis 95 % Gew.-% Trägerstoffe, insbesondere Maltodextrin und/oder Stärke, 5 bis 40 % Hilfsstoffe, bevorzugt natürliche oder künstliche Polysaccharide und/oder Pflanzengummen wie modifizierte Stärken oder Gummi Arabicum enthalten.

Gemäß einer weiteren bevorzugten Ausführungsform werden zur Herstellung erfindungsgemäßer Zubereitungen die Verbindung bzw. die Verbindungen der Formel (A) und gegebenenfalls andere Bestandteile der erfindungsgemäßen Zubereitung zunächst in Emulsionen, in Liposomen, z.B. ausgehend von Phosphatidylcholin, in Microsphären, in Nanosphären oder auch in Kapseln, Granulaten oder Extrudaten aus einer für Lebens- und Genussmittel geeigneten Matrix, z.B. aus Stärke, Stärkederivaten (z.B. modifizierte Stärke), Cellulose oder Cellulosederivaten (z.B. Hydroxypropylcellulose), anderen Polysacchariden (z.B. Dextrin, Alginat, Curdlan, Carageenan, Chitin, Chitosan, Pullulan), natürlichen Fetten, natürlichen Wachsen (z.B. Bienenwachs, Carnaubawachs), aus Proteinen, z.B. Gelatineoder sonstigen Naturprodukten (z.B. Schellack) eingearbeitet. Dabei können je nach Matrix die Produkte durch Sprühtrocknung, Sprühgranulation, Schmelzgranulation, Koazervation, Koagulation, Extrusion, Schmelzextrusion, Emulsionsverfahren, Beschichtung (Coating) oder andere geeignete Verkapselungsverfahren und gegebenenfalls eine geeignete Kombination der vorgenannten Verfahren erhalten werden. In einem weiteren bevorzugten Herstellungsverfahren für eine erfindungsgemäße Zubereitung werden die Verbindung bzw. die Verbindungen der Formel (A) zunächst mit einem oder mehreren geeigneten Komplexbildnern, beispielsweise mit Cyclodextrinen oder Cyclodextrinderivaten, bevorzugt alpha- oder beta-Cyclodextrin, komplexiert und in dieser komplexierten Form eingesetzt.

Besonders bevorzugt ist eine erfindungsgemäße Zubereitung, bei der die Matrix so gewählt ist, dass die erfindungsgemäßen Verbindungen der Formel (A) verzögert von der Matrix freigegeben werden, so dass eine langanhaltende Wirkung erzielt wird. Besonders bevorzugt ist insoweit eine Fett-, Wachs-, Polysaccharid- oder Proteinmatrix.

Als weitere Bestandteile für erfindungsgemäße, der Ernährung oder dem Genuss dienende Zubereitungen können übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nichtproteinogene Aminosäuren und verwandte Verbindungen (z.B. γ-Aminobuttersäure, Taurin), Peptide (z.B. Glutahthion), native oder prozessierte Proteine (z.B. Gelatine), Enzyme (z.B. Peptidasen), Nukleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin, Amarogentin, Humolone, Lupolone, Catechine, Tannine), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigentien.

Erfindungsgemäße Zahnpflegemittel (als Basis für der Mundpflege dienende Zubereitungen), die eine oder mehrere Verbindung der Formel (A) bzw. eine Mischung dieser Verbindungen enthalten, umfassen im Allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite^{®}, Süßstoffe, wie z.B. Saccharin, Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin und Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Erfindungsgemäße Kaugummis (als weiteres Beispiel für der Mundpflege dienende Zubereitungen), welche eine oder mehrere Verbindungen der Formel (A) bzw. Mischungen dieser Verbindungen enthalten, umfassen im allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkoholen, Geschmackskorrigenzien für unangenehme Geschmackseindrücke, andere Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Aromen und Stabilisatoren oder Geruchskorrigentien.

Nachfolgend wird die Erfindung anhand von Beispielen näher erläutert. Sofern nicht anders angegeben, beziehen sich alle Angaben auf das Gewicht.

Die im Folgenden angegebenen Retentions-Indices (RI) beziehen sich auf folgende GC-Bedingungen: Säule: 20m DB-Wax, Innendurchmesser: 0,18 mm; Filmdicke df = 0,18 µm; Temperaturprogramm 60-9-220°C, Kaltaufgabesystem (KAS).

### Beispiel 1: Parfümöl mit bzw. ohne 2-Ethoxymethyl-3-isopropenyl-1-methylcyclopenten (1 b) ("Rosenduft"-Thema)

| Riechstoff | A | B |
|---|---|---|
| | Gew.-teile | Gew.-teile |
| Undecanal (Aldehyd C11) | 5,0 | 5,0 |
| Benzophenon | 20,0 | 20,0 |
| Citronellol | 110,0 | 110,0 |
| 1-Cyclohexyl-ethyl-crotonat | 30,0 | 30,0 |
| Decenol trans-9 | 5,0 | 5,0 |
| Dihydromyrcenol | 40,0 | 40,0 |
| Diphenyloxid | 10,0 | 10,0 |
| Ethylvanillin, 10% in DPG | 20,0 | 20,0 |
| Eugenol | 10,0 | 10,0 |
| Frambinon, 10% in DPG | 10,0 | 10,0 |
| Geraniol | 60,0 | 60,0 |
| 15-Pentadecenolid | 40,0 | 40,0 |
| 8-Cyclohexadecenon | 40,0 | 40,0 |
| Hexenylsalicylat cis-3 | 30,0 | 30,0 |
| Ionon Beta | 20,0 | 20,0 |
| 1-(2,4,4-Trimethylcyclohex-2-en-1-yl) but-2-en-1-on-en-1-yl) | 5,0 | 5,0 |
| Cyclohexadecanon | 20,0 | 20,0 |
| 4-tert.butyl-α-methyldihydrocinnamaldehyd | 80,0 | 80,0 |
| Linalool | 80,0 | 80,0 |
| 2,2-Dimethyl-3-(3-methylphenyl)-propanol | 20,0 | 20,0 |
| Methylphenylacetat | 10,0 | 10,0 |
| Palmarosaöl | 5,0 | 5,0 |
| Phenoxyethylalkohol | 30,0 | 30,0 |
| Phenylethylalkohol | 200,0 | 200,0 |
| Tetrahydrolinalool | 40,0 | 40,0 |
| 2,4-Dimethylcyclohex-3-en-1-carbaldehyd | 5,0 | 5,0 |
| 1,3-Dimethyl-3-phenylbutylacetat | 5,0 | 5,0 |
| 1',1',5',5'-Tetramethylhexahydro-spiro[1,3-dioxolan-2,8'(5'H)-2H-2,4a-methanonaphthalin | 5,0 | 5,0 |
| Zimtalkohol | 5,0 | 5,0 |
| Dipropylenglycol | 40,0 | 10,0 |
| 2-Ethoxymethyl-3-isopropenyl-1-methylcyclopenten (1 b) | - | 30,0 |
| Summe: | 1000,0 | 1000,0 |

| | | |
|---|---|---|
| DPG: Dipropylenglycol | | |

Die beiden Parfümöle A (nicht erfindungsgemäß) und B (erfindungsgemäß) wurden in einen Weichspüler eingearbeitet und anschließend aus dem Weichspüler heraus geruchlich beurteilt (0,8 Gew.-% Dosierung). Der Effekt lässt sich nach Meinung der Parfümeure wie folgt beschreiben: das erfindungsgemäße Parfümöl B ist voller, runder, frischer, blumiger (insbesondere die Noten Flieder und Osmanthus), fruchtiger (insbesondere Aprikose), damasconiger, rosiger, strahlender.

### Beispiel 2: Darstellung der Verbindung der Formel (1 b) (2-Ethoxymethyl-3-isopropenyl-1-methylcyclopenten) in vier Stufen

### Stufe a):

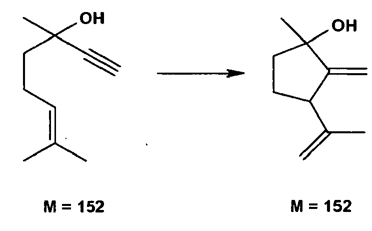

470 g (3,1 mol) Dehydrolinalool (DHL) wurden bei 185-192°C unter Rückfluss erhitzt. Die Umsetzung wurde gaschromatographisch verfolgt. Es wurde solange erhitzt, bis der Restgehalt an DHL (RI 1700) ca. 1 % betrug (ca. 12 - 15 h). Anschließend wurde abgekühlt. Die Rohausbeute betrug 464,9 g (98,9 %), welche das gewünschte Produkt in Form von 2 Isomeren im Verhältnis: 56 : 44 enthielt (RI 1520 bzw. 1580). Dieses Rohprodukt wurde direkt in die Stufe b) eingesetzt.

### Stufe b):

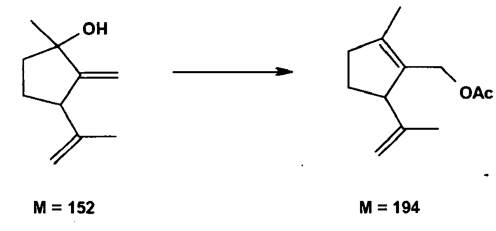

Man legte ein Gemisch aus 342,6 g Acetanhydrid, 0,3 g Wolframsäure und 4,9 g Natriumacetat vor und erhitzte auf 135°C. Anschließend wurde bei dieser Temperatur 464,9 g (3,06 mol) Rohprodukt aus Stufe a) innerhalb von 30 min. zugetropft und 2 h unter Rückfluss erhitzt. Es wurde auf 80°C abgekühlt und mit 500 mL Wasser hydrolysiert. Anschließend wurde zweimal mit je 300 mL Hexan extrahiert. Die vereinigten organischen Phasen wurden nacheinander mit 200 mL Wasser, 200 mL Bicarbonatlösung und erneut 100 mL Wasser neutral gewaschen. Es wurde über Natriumsulfat getrocknet und das Lösungsmittel am Rotationsverdampfer abgezogen.

Rohausbeute: 579 g (97 %).

Anschließend wurde an einer 20 cm Vigreuxkolonne im Vakuum fraktioniert. Destillationsbedingungen: 80-90°C / 4 mbar Ausbeute: 341,3 g (78%). Die GC-Reinheit des gewünschten Produkts (RI 1658) lag bei 96 %, daneben wurden 2% Dehydrolinalylacetat (RI 1685) erhalten.

Stufe c): 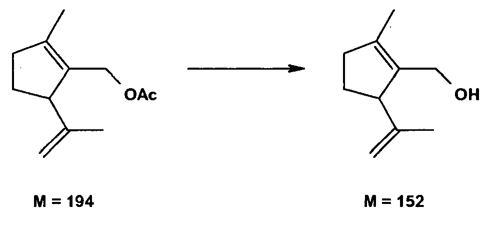 Eine Mischung aus 30 g Natriummethylat in 1000 mL Methanol wurde vorgelegt. Anschließend wurden innerhalb von 15 min. 341 g des Acetats aus Stufe b) zugetropft und zwei Tage bei Raumtemperatur (ca. 20°C) stehengelassen. Die Reaktion wurde gaschromatographisch verfolgt. Falls noch Edukt-Acetat vorliegt, wird kurz erwärmt. Anschließend wurde das Methanol am Rotationsverdampfer (40°C, 30 mbar) weitgehend abgezogen und die organische Phase mit 500 mL Methyl-tert.butylether (MTBE) aufgenommen. Es wurde dreimal mit je 300 mL Wasser gewaschen, über Natriumsulfat getrocknet und das Lösungsmittel am Rotationsverdampfer abgezogen.

### Rohausbeute: 268,9 g

Anschließend wurde an einer 20 cm Vigreuxkolonne fraktioniert.

### Ausbeute: 243,7 g (90 %), Destillationsbedingungen: Kp = 85°C/3 mbar

GC-Reinheit: 96% Alkohol, 2 % Dehydrolinalool

Stufe d): 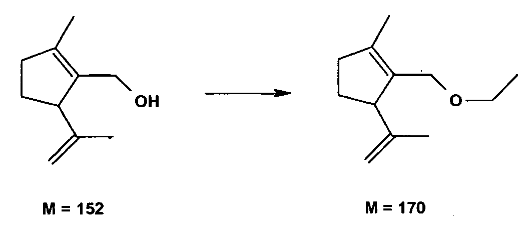 Es wurde ein Gemisch aus 800 g NaOH 50%, 1600 mL Toluol und 128,5 g Tetrabutylammoniumbromid (50% in Wasser) vorgelegt. Bei Raumtemperatur (ca. 20 °C) wurden 243 g Alkohol aus Stufe c) zudosiert und unter starkem Rühren auf 50°C erhitzt. Anschließend werden 770 g Diethylsulfat innerhalb von 2,5 h zudosiert. Es wurde 10 h bei 50°C gerührt. Zur Umsatzkontrolle werden Proben gezogen (mit Ammoniak überschüssiges Diethylsulfat zerstören). Es blieben ca. 10% Alkohol unumgesetzt. Anschließend werden 200 mL konz. Ammoniak zugetropft und 4 h bei 40°C gerührt. Man kühlt ab, trennt die Phasen und wäscht mit Wasser neutral. Am Rotationsverdampfer wird bei 40°C und 30 mbar eingeengt.

### Rohausbeute: 286,0 g

Es wurde über eine 20 cm Vigreuxkolonne fraktioniert.

### Ausbeute: 231,4 g (85 %), Destillationsbedingungen: 88-95°C / 25 mm

Alle Fraktionen mit einem Gehalt des Ethers >96% werden zusammengegeben.

### RI (Verbindung 1b): 1390

RI (Nebenprodukt Dehydrolinalylethylether): 1408 (∼2-3%)
¹H-NMR (400 MHz, CDCl₃): δ = 1,18 ppm (t), 1,60 (dd), 1,65 (m), 1,74 (m), 2,04 (m), 2,32 (m), 3,39 (dq), 3,46 (dq), 3,46 (m), 3,72 (d), 4,02 (d), 4,70 (m).

IR (Film): 890, 1100, 1370, 1450, 1650, 2850, 2950, 3000, 3180 cm⁻¹
MS (70 eV): 180, 165 (M-CH₃), 151 (M-C₂H₅), 136 (M-C₂H₄O), 134 (M-C₂H₅OH), 119 (100%, M-CH₃ - C₂H₅OH) 91, 79, 41

### Beispiel 3: Darstellung von Verbindungen der Formel (A) in zwei Stufen

### Beispiel 3.1: Darstellung der Verbindung der Formel 1b (2-Ethoxymethyl-3-isopropenyl-1-methylcyclopenten)

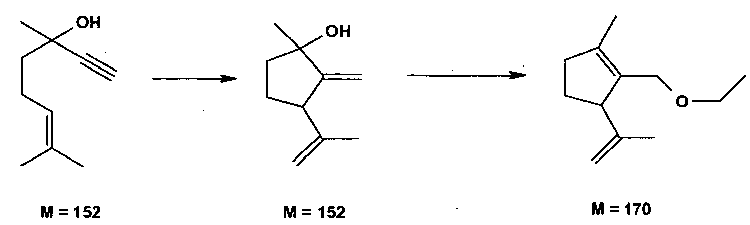

488 g Dehydrolinalool wurden 15 Stunden unter Rückfluss erhitzt. Anschließend wurde bei 3-4 mbar und einer Sumpftemperatur von 120°C vom Rückstand abdestilliert, wobei 488 g Destillat erhalten werden. Hierzu gab man 675 g Triethylorthoformiat, 1380 g Ethanol und 7,2 g p-Toluolsulfonsäure und erhitzte 8 Stunden unter Rückfluss. Nach Reaktionsende wurde Ethanol abdestilliert und jeweils 500 g MTBE und Wasser hinzugefügt. Nach Phasentrennung wurde die organische Phase mit Wasser neutral gewaschen und der MTBE abdestilliert. Der verbleibende Rückstand wurde bei einer Manteltemperatur von 110°C und einem Vakuum von 1 mbar über einen Dünnschichtverdampfer destilliert. Es resultierten 340 g Destillat, die anschließend fraktioniert wurden. Es wurden 217 g Produkt (Kp_{3mbar} = 62°C) mit einem Gehalt von 98,7% erhalten. Dieses entsprach einer Ausbeute von 40% d.Th.

### Beispiel 3.2: Darstellung einer Mischung der Verbindungen 2aE/2aZ bzw. 2bE/2bZ:

Analog zu Beispiel 3.1 wurden die (E)/(Z)-Isomerenmischungen 2aE/2aZ sowie 2bE/2bZ hergestellt. Das (E)/(Z)-Isomerenverhältnis lag nach Destillation jeweils bei circa 55 : 35.

Die hervorragenden anwendungstechnischen Eigenschaften von 2-Ethoxymethyl-3-isopropenyl-1-methylcyclopenten 1b werden nachfolgend an einigen Beispielen verdeutlicht.

### Beispiel 4: Eau de Toilette (EDT) - Beispiel 5: Antiperspirant (AP) - Stick

Das Vermitteln einer fruchtig, blumig, osmanthusartigen Note in einem Eau de Toilette (EDT) und in einem Antiperspirant (AP) - Stick wurde im Vergleich zu Dimethylbenzylcarbinylbutyrat (DMBCB) (Referenz) von einem Panel auf einer Skala von 0 (keine Note) bis 6 (sehr stark fruchtig, blumige, osmanthusartige-Note) bewertet:

| EDT | 2-Ethoxymethyl-3-isopropenyl-1-methylcyclopenten 1b | DMBCB |
|---|---|---|
| Nach 1 Stunde⁺ | 2,9 | 1,4 |
| Nach 3 Stunden⁺ | 2,1 | 0,9 |
| Nach 16 Stunden⁺ | 0,9 | 0,8 |

| | | |
|---|---|---|
| ⁺: Zeit nach Aufbringen einer definierten Menge auf einen Papier-Riechstreifen | | |

Die EDT-Rezeptur bestand aus 80 Gew.-% Ethanol, 19,7 Gew.-% Wasser und 0,3 Gew.-% 2-Ethoxymethyl-3-isopropenyl-1-methylcyclopenten 1b bzw. DMBCB.

| AP-Stick | 2-Ethoxymethyl-3-isopropenyl-1-methylcyclopenten 1b | DMBCB |
|---|---|---|
| Vom Stick | 2,4 | 1,9 |
| 1 Stunde nach Auftragen auf die Haut | 2,8 | 1,1 |

Die erfindungsgemäße Verbindung erwies sich demnach als überlegen.

### Beispiel 6: Blooming

Bezüglich des Bloomings (Geruch aus einer wässrigen Tensid-Lösung, hier: wässrige Lösung eines Weichspülers) wurde 2-Ethoxymethyl-3-isopropenyl-1-methylcyclopenten 1b in einem direkten Performance-Test mit DMBCB verglichen (Panel-Bewertung 1-9):

| Blooming | 2-Ethoxymethyl-3-isopropenyl-1-methylcyclopenten 1b | DMBCB |
|---|---|---|
| | 6 | 4 |

Die erfindungsgemäße Verbindung erwies sich als überlegen.

### Beispiel 7: Diffusivität

Diffusivität ist definiert als der Wert der geruchlichen Wahrnehmung in einer bestimmten Zeit und einer bestimmten Entfernung. Je höher der Wert, desto schneller und stärker wird die Substanz wahrgenommen.

Die Skala ist definiert von 1 = kein Impact nach 3 min. bis 9 = hoher Impact nach 10 sec.

Diffusivität wird bestimmt durch Riechen einer bestimmten Menge eines Materials in einer Petrischale bei einem bestimmten Abstand zu dem jeweiligen Panelisten. Die Schale wird durch ein Signal geöffnet, die Zeit gestoppt und die Intensität nach der Zeit des Impactes beurteilt.

| Diffusivität | 2-Ethoxymethyl-3-isopropenyl-1-methylcyclopenten 1b | DBMBCB |
|---|---|---|
| | 5,9 | 3,8 |

Die erfindungsgemäße Verbindung erwies sich als überlegen.

### Beispiel 8: Antiperspirant-Stick

| Komponente | Gew.-% | Gew.-% |
|---|---|---|
| Phenyl Trimethicon (SilCare TM Silicone 15 M 50) | 13,50 | 13,50 |
| Cetearyl Alkohol | Ad 100 | Ad 100 |
| Cetiol CC (Dicaprylyl Carbonat) | 13,50 | 13,50 |
| Stearinsäure | 3,50 | 3,50 |
| PEG-40-Hydriertes Rizinusöl (Emulsogen TM HCO 040) | 4,10 | 4,10 |
| PEG-8 Distearate (Cithrol 4 DS) | 4,10 | 4,10 |
| Petrolatum | 6,90 | 6,90 |
| Aluminiumchlorohydrat | 14,00 | 13,80 |
| Aluminium Zirconium Trichlorohydrex Gly | 20,00 | 19,50 |
| 2,2-Dimethyl-3-phenylpropanol (Muguetalkohol) | - | 0,25 |
| Ethylhexylglycerin (Octoxyglycerin) | - | 0,30 |
| 2-Methyl-5-phenylpentan-1-ol (Rosaphen) | 0,30 | - |
| Parfümöl B (erfindungsgemäß) aus Beispiel 1 | 0,80 | 1,00 |

### Beispiel 9: Deostifte

| Komponente | A | B |
|---|---|---|
| | Gew.-% | Gew.-% |
| Natriumstearat | 7,00 | 8,00 |
| Natriumpalmitat | 1,00 | - |
| 1,2-Propylenglycol | 42,00 | 45,00 |
| 1,2-Butylenglycol | 3,00 | - |
| 2-Methyl-5-phenylpentan-1-ol (Rosaphen) | 0,50 | 0,25 |
| 2-Butyloctansäure | - | 0,50 |
| 2-Hexyldecansäure | 0,10 | - |
| Polyethylenglycol(25)cetearylether | 3,00 | 3,00 |
| Ethanol | 20,00 | 20,00 |
| Farnesol | - | 0,25 |
| Triclosan® (5-Chlor-2-(2,4-dichlorphenoxy)phenol) | 0,30 | - |
| Parabene (Mischung aus Methyl-, Ethyl-, Propyl-, Butyl-, Isobutylparaben) | 0,30 | - |
| 1,2-Hexandiol / 1,2-Octandiol (1:1) | - | 0,50 |
| 1,2-Pentandiol | 0,50 | - |
| Parfümöl B (erfindungsgemäß) aus Beispiel 1 | 1,00 | 0,90 |
| Wasser | Ad 100 | Ad 100 |

### Beispiel 10: Air-freshener in Gelform

5 g Accurel (poröses Homo-Polypropylen Pulver mit 75% Hohlraumanteil, ein Produkt der Akzo Nobel Faser AG, Obernburg, Deutschland) werden mit 15 g Parfümöl aus Beispiel 1 durch Mischen der beiden Komponenten unter Vakuum beladen. Das resultierende Pulver wird anschließend bei Normaldruck mit 4,5 g Wasser verrührt (Mix 1). In einem anderen separaten Gefäß werden 2,5 g Carragheen, 0,3 g Chloracetamid und 0,5 g Calciumchlorid-Dihydrat in 62 g Wasser unter Erwärmen bis auf etwa 75 °C gelöst. In diese Lösung wird unter Rühren Mix 1 eingebracht und homogenisiert. Die resultierende, noch warme Mischung wird in die gewünschte Form (Kugeln, Halbkugeln, Kissen, Zylinder, Quader, Würfel, Muscheln oder ähnliche) gegossen. Nach dem Abkühlen auf etwa 20°C werden Raumerfrischer in Gelform erhalten, deren Beladung an Parfümöl bei etwa 20 Gew.-% liegt.

### Beispiel 11: Shampoo

Parfümölkompositionen A und B aus Beispiel 1 wurden in einer Dosierung von je 0,2 Gew.-% separat in eine Shampoo-Grundmasse der folgenden Rezeptur eingearbeitet:

| | |
|---|---|
| Natriumlaurylethersulfat | 12% |
| (z.B. Texapon NSO, Fa. Cognis Deutschland GmbH) | |
| Cocamidopropylbetain | 2% |
| (z.B. Dehyton K, Fa. Cognis Deutschland GmbH) | |
| Natriumchlorid | 1,4% |
| Citronensäure | 1,3% |
| Phenoxyethanol, Methyl-, Ethyl-, Butyl-, | |
| und Propylparaben | 0,5% |
| Pfirsichriechstoffmischung umfasend gamma-Undecalacton | 0,5% |
| Wasser | 82,3% |

Der pH-Wert der Shampoo-Grundmasse lag bei etwa 6. Hieraus wurden 100 mL einer 20 Gew.%-igen wässrigen Shampoo-Lösung hergestellt. In dieser Shampoo-Lösung wurden zwei Haarsträhnchen gemeinsam für 2 Minuten gewaschen und anschließend 20 Sekunden unter fließendem handwarmen Wasser gespült. Eine Haarsträhne wurde nass in Aluminiumfolie eingepackt und die zweite Haarsträhne mit einem Fön getrocknet. Beide Haarsträhnen wurden von einem Panel geruchlich beurteilt. Das Shampoo und die Haarsträhne mit Parfümöl B wurde als voller, runder, frischer, blumiger (insbesondere die Noten Flieder und Osmanthus), fruchtiger (insbesondere Aprikose), damasconiger, rosiger, strahlender beschrieben.

### Beispiel 12: Weichspüler

Die Parfümölkompositionen A und B aus Beispiel 1 wurden je in einer Dosierung von 0,7 Gew.-% separat in eine Weichspüler-Grundmasse folgender Zusammensetzung eingearbeitet:

| | |
|---|---|
| Quarternäres Ammoniummethosulfat (Esterquat), ca. 90% | 5,5% |
| (z.B. Rewoquat WE 18, Fa. Witco Surfactants GmbH) | |
| Alkyldimethylbenzylammoniumchlorid, ca. 50% | 0,2% |
| (z.B. Preventol R50, Fa. Bayer) | |
| Farblösung, ca. 1%-ig | 0,3% |
| Wasser | 94,0% |

Der pH-Wert des Weichspüler-Grundmasse lag im Bereich 2 - 3. Zwei Stofflappen wurden mit 370 g einer 1%-igen wässrigen Weichspüler-Lösung in einer Linetest-Maschine im Weichspülprogramm 30 Minuten bei 20°C gespült.
Die Lappen wurden ausgewrungen und anschließend 20 Sekunden geschleudert. Ein Lappen wurde nass eingeschweißt, und einer zum Trocknen aufgehängt. Anschließend wurden beide Lappen durch ein Panel geruchlich beurteilt. Der Lappen mit Parfümöl B wurde als voller, runder, frischer, blumiger (insbesondere die Noten Flieder und Osmanthus), fruchtiger (insbesondere Aprikose), damasconiger, rosiger, strahlender beschrieben.

### Beispiel 13: Waschpulver

2-Ethoxymethyl-3-isopropenyl-1-methylcyclopenten der Formel 1b und Parfümölkomposition B aus Beispiel 1 wurden separat in einer Dosierung von je 0,3 Gew.-% in eine Waschpulver-Grundmasse der folgenden Rezeptur eingearbeitet:

| | |
|---|---|
| Lineares Na-Alkylbenzolsulfonat | 8,8 % |
| Ethoxylierter Fettalkohol C12-18 (7 EO) | 4,7 % |
| Na-Seife | 3,2 % |
| Entschäumer | |
| DOW CORNING(R) 2-4248S | |
| POWDERED ANTIFOAM, | |
| Silikonöl auf Zeolith als Trägermaterial | 3,9 % |
| Zeolith 4A | 28,4% |
| Na-Carbonat | 11,6 % |
| Na-Salz eines Copolymers aus Acryl- | |
| und Maleinsäure (Sokalan CP5) | 2,4 % |
| Na-Silicat | 3,0 % |
| Carboxymethylzellulose | 1,2 % |
| Dequest 2066 | 2,8 % |
| ([[(Phosphonomethyl)imino]bis[(ethylennitrilo)bis | |
| (methylen)]]tetrakis-phosphonsäure, Natriumsalz) | |
| Optischer Aufheller | 0,2 % |
| Na-Sulfat | 6,6 % |
| Protease | 0,4 % |
| Natriumperborattetrahydrat | 21,8% |
| TAED | 1,0 % |

Zwei Stofflappen wurden mit 370 g einer 1%-igen wässrigen Waschpulverlauge (der pH-Wert der Waschpulverlauge liegt deutlich im basischen Bereich) in einer Linetest-Maschine im Hauptwaschgang 45 Minuten bei 60°C gewaschen. Die Lappen wurden zunächst 5 Minuten mit kaltem Wasser gespült, ausgewrungen und anschließend 20 Sekunden geschleudert. Ein Lappen wurde nass eingeschweißt, und einer zum Trocknen aufgehängt.

### Beispiel 14: Blumig-frisches Fruchtaroma

| Bestandteil | Gewichtsteile |
|---|---|
| Vanillin | 10,0 |
| Ethylacetat | 150,0 |
| Ethylbutyrat | 200,0 |
| Ethylpropionat | 50,0 |
| 2,6-Nonadienol, 2 % in Alkohol | 2,0 |
| cis-6-Nonenal, 1 % in Alkohol | 0,2 |
| Methyldihydrojasmonat | 7,0 |
| Furaneol 15 % in 1,2-Propylenglycol | 30,0 |
| beta-lonon | 0,2 |
| Isoamylacetat | 60,0 |
| Isoamylalkohol | 8,0 |
| Isobutylacetat | 100 |
| (2E,8Z)-Undecadiensäureethylester | 20 |
| (2E,6Z)-Nonadienal, 1 % in 1,2-Propylenglycol | 2,0 |
| 6Z-Nonenol | 2,0 |
| 2-Ethoxymethyl-3-isopropenyl-1-methylcyclopenten der Formel (1 b) | 20 |
| 1,2-Propylenglycol | 338,6 |

### Beispiel 15: Carbonisiertes Erfrischungsgetränk

| Bestandteil | kg | Liter |
|---|---|---|
| Zuckersirup, 65 % Feststoffanteil | 153,850 | 116,55 |
| Zitronensäurelösung 50 % | 3,000 | 2,460 |
| Aroma aus Beispiel 14 | 0,200 | 0,250 |
| Carbonisiertes Wasser | 880,740 | 880,740 |
| Summe Zubereitung | 1037,790 | 1000,000 |

## Patentansprüche

1. Verbindung der Formel (A) 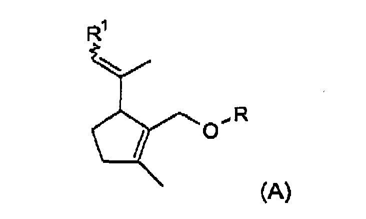 wobei unabhängig voneinander für die Gruppen R und R¹ gilt:
R ist Methyl oder Ethyl, und
R¹ ist Wasserstoff oder Methyl, wobei die geschlängelte Linie anzeigt, dass für
R¹ = Methyl die zugehörige Doppelbindung (E)- oder (Z)-konfiguriert ist.

2. Verbindung nach Anspruch 1, wobei gilt:
R ist Ethyl
und
R¹ ist Wasserstoff.

3. Verwendung einer Verbindung nach Anspruch 1 oder 2 als Riech- und/oder Aromastoff.

4. Verwendung nach Anspruch 3 zum Vermitteln, Modifizieren und/oder Verstärken einer fruchtigen (insbesondere Aprikose), blumigen (insbesondere Flieder), süßen und osmanthusartigen Geruchs- oder Geschmacksnote.

5. Verwendung einer Verbindung nach Anspruch 1 oder 2 zum Erhöhen des über einer tensidhaltigen wässrigen Lösung wahrgenommenen Geruches anderer Riechstoffe.

6. Artikel umfassend eine oder mehrere Verbindungen der Formel (A) gemäß Anspruch 1 oder 2.

7. Artikel nach Anspruch 6, umfassend oder bestehend aus zwei oder mehr Verbindungen der Formel (A) gemäß Anspruch 1 oder 2,

8. Artikel nach Anspruch 6, umfassend oder bestehend aus einer ersten und einer zweiten Verbindung der Formel (A) gemäß Anspruch 1, wobei in der ersten und der zweiten Verbindung R identisch ist, R¹ Methyl ist und die zugehörige Doppelbindung der ersten Verbindung (E)-konfiguriert und die der zweiten Verbindung (Z)-konfiguriert ist.

9. Artikel nach einem der Ansprüche 6 bis 8, umfassend eine Gesamtmenge an Verbindungen der Formel (A) gemäß einem der Ansprüche 1 oder 2, die ausreicht, um eine, zwei, drei oder sämtliche Geruchs- oder Geschmacksnoten aus der Gruppe bestehend aus fruchtig (insbesondere Aprikose), blumig (insbesondere Flieder), süß und osmanthusartig zu vermitteln, zu modifizieren und/oder zu verstärken.

10. Artikel nach einem der Ansprüche 6 bis 9, wobei der Artikel eine Riech- oder Aromastoffkomposition ist, umfassend eine oder mehrere Verbindungen der Formel (A) gemäß einem der Ansprüche 1 oder 2 sowie einen oder mehrere weitere Riech- bzw. Aromastoffe.

11. Artikel nach einem der Ansprüche 6 bis 10, weiter umfassend einen Träger oder ein Substrat, der bzw. das in direktem Kontakt mit der oder den Verbindungen der Formel (A) bzw. der Riech- oder Aromastoffkomposition steht.

12. Riech- oder Aromastoffkomposition nach einem der Ansprüche 10 bis 11, umfassend eine Gesamtmenge von Verbindungen der Formel (A) gemäß Anspruch 1 oder 2 im Bereich von 0,001 bis 70 Gew.-%, vorzugsweise 0,05 bis 50 Gew.-% und besonders bevorzugt 0,5 bis 25 Gew.-%, bezogen auf die Gesamtmenge der Riech- bzw. Aromastoffkomposition.

13. Verfahren zum Vermitteln. Modifizieren und/oder Verstärken eines Geruchs oder Geschmacks, wobei eine Menge (a) einer oder mehrerer Verbindungen der Formel (A) gemäß Anspruch 1 oder 2 oder (b) einer Riech- oder Aromastoffkomposition nach einem der Ansprüche 10 bis 12 mit einem Erzeugnis in Kontakt gebracht oder gemischt wird.

14. Verfahren nach Anspruch 13 zum Vermitteln, Modifizieren und/oder Verstärken eines Geruchs oder Geschmacks mit einer, zwei, drei oder sämtlichen der Noten fruchtig (insbesondere Aprikose), blumig (insbesondere Flieder), süß und osmanthusartig.

15. Verfahren zum Modifizieren und/oder Verstärken eines Geruchs oder Geschmacks mit einer, zwei, drei oder sämtlichen der Noten fruchtig (insbesondere Aprikose), blumig (insbesondere Flieder), süß und osmanthusartig, mit folgendem Schritt:
- Vermischen
einer Menge an einer oder mehreren Verbindungen der Formel (A) gemäß Anspruch 1 oder 2
mit
einem oder mehreren sonstigen Riech- oder Aromastoffen mit einer, zwei, drei oder sämtlichen der Noten fruchtig (insbesondere Aprikose), blumig (insbesondere Flieder), süß und osmanthusartig,
wobei die eingesetzte Menge der einen oder mehreren Verbindungen der Formel (A) gemäß Anspruch 1 oder 2 ausreicht, den Geruchs- bzw. Geschmackseindruck der sonstigen Riech- bzw. Aromastoffe, die eine, zwei, drei oder sämtliche der Noten fruchtig (insbesondere Aprikose), blumig (insbesondere Flieder), süß und osmanthusartig verursachen, sensorisch zu modifizieren und/oder zu verstärken.

16. Verfahren zur Herstellung einer Verbindung der Formel (A) gemäß Anspruch 1 oder 2, mit folgenden Schritten:
(i) Bereitstellen oder Herstellen einer Verbindung der Formel (C) 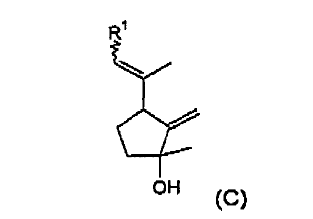 sowie
(ii) Umsetzen der Verbindung der Formel (C) in einem oder mehreren Schritten, so dass eine Allylumlagerung und eine Veretherung zur Verbindung der Formel (A) resultiert.

17. Verfahren zur Herstellung eines Artikels nach einem der Ansprüche 6 bis 12, mit folgendem Schritt:
- Vermischen einer oder mehrer Verbindungen der Formel (A) gemäß Anspruch 1 oder 2 mit weiteren Bestandteilen, wobei eine Menge einer oder mehrer Verbindungen der Formel (A) eingesetzt wird, die ausreicht, um in dem Artikel eine Geruchs- oder Geschmacksnote zu vermitteln, zu modifizieren und/oder zu verstärken.

## Claims

1. Compound of the formula (A) 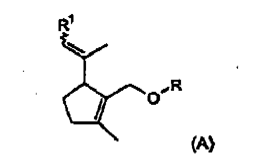 wherein, independently of each other for the groups R and R¹;
R is methyl or ethyl and
R¹ is hydrogen or methyl, wherein the wavy line indicates that for R¹ = methyl the associated double bond is in the (E) or (Z) configuration.

2. Compound according to claim 1, wherein:
R is ethyl
and
R¹ is hydrogen.

3. Use of a compound according to claim 1 or 2 as an odoriferous and/or aroma substance.

4. Use according to claim 3 for imparting, modifying and/or intensifying a fruity (in particular apricot), flowery (in particular lilac), sweet and osmanthus-like olfactory or flavour note.

5. Use of a compound according to claim 1 or 2 for increasing the smell of other odoriferous substances perceived via a surfactant-containing aqueous solution.

6. Article comprising one or more compounds of the formula (A) according to claim 1 or 2.

7. Article according to claim 6, comprising or consisting of two or more compounds of the formula (A) according to claim 1 or 2.

8. Article according to claim 6, comprising or consisting of a first and a second compound of the formula (A) according to claim 1, wherein in the first and the second compound R is identical, R¹ is methyl and the associated double bond of the first compound is in the (E) configuration and that of the second compound is in the (Z) configuration.

9. Article according to one of claims 6 to 8, comprising a total amount of compounds of the formula (A) according to one of claims 1 or 2 which is sufficient to impart, to modify and/or to intensify one, two, three or all of the olfactory or flavour notes from the group consisting of fruity (in particular apricot), flowery (in particular lilac), sweet and osmanthus-like.

10. Article according to one of claims 6 to 9, wherein the article is an odoriferous or aroma composition comprising one or more compounds of the formula (A) according to one of claims 1 or 2 and one or more further odoriferous or aroma substances.

11. Article according to one of claims 6 to 10, furthermore comprising a carrier or a substrate which is in direct contact with the compound or compounds of the formula (A) or the odoriferous or aroma substance composition.

12. Odoriferous or aroma substance composition according to one of claims 10 to 11, comprising a total amount of compounds of the formula (A) according to claim 1 or 2 in the range of from 0.001 to 70 wt.%, preferably 0.05 to 50 wt.% and particularly preferably 0.5 to 25 wt.%, based on the total amount of the odoriferous or aroma substance composition.

13. Method for imparting, modifying and/or intensifying a smell or flavour, wherein an amount (a) of one or more compounds of the formula (A) according to claim 1 or 2 or (b) of an odoriferous or aroma substance composition according to one of claims 10 to 12 is brought into contact or mixed with a product.

14. Method according to claim 13 for imparting, modifying and/or intensifying a smell or flavour with one, two, three or all of the notes of fruity (in particular apricot), flowery (in particular lilac), sweet and osmanthus-like.

15. Method for modifying and/or intensifying a smell or flavour with one, two, three or all of the notes of fruity (in particular apricot), flowery (in particular lilac), sweet and osmanthus-like, with the following step:
- mixing
of an amount of one or more compounds of the formula (A) according to claim 1 or 2
with
one or more other odoriferous or aroma substances with one, two, three or all of the notes of fruity (in particular apricot), flowery (in particular lilac), sweet and osmanthus-like,
wherein the amount of the one or more compounds of the formula (A) according to claim 1 or 2 employed is sufficient to sensorially modify and/or to intensify the olfactory or flavour impression of the other odoriferous or aroma substances which cause one, two, three or all of the notes of fruity (in particular apricot), flowery (in particular lilac), sweet and osmanthus-like.

16. Process for the preparation of a compound of the formula (A) according to claim 1 or 2, with the following steps:
(i) provision or preparation of a compound of the formula (C) 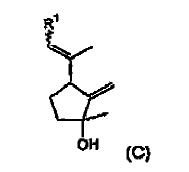 and
(ii) reaction of the compound of the formula (C) in one or more steps such that an allyl rearrangement and an etherification to give the compound of the formula (A) results.

17. Process for the production of an article according to one of claims 6 to 12, with the following step:
- mixing of one or more compounds of the formula (A) according to claim 1 or 2 with further constituents, wherein an amount of one or more compounds of the formula (A) which is sufficient to impart, to modify and/or to intensify an olfactory or flavour note in the article is employed.

## Revendications

1. Composé de formule (A) 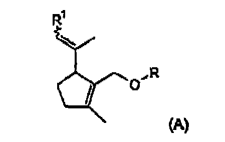 les groupes R et R¹ représentant, indépendamment l'un de l'autre:
R, le méthyle ou l'éthyle et
R¹ l'hydrogène ou le méthyle, la ligne sinueuse indiquant que, pour R¹ = méthyle, le double liaison associée est configurée (E) ou (Z).

2. Composé selon la revendication 1,
R étant l'éthyle
et
R¹ étant l'hydrogène.

3. Utilisation d'un composé selon la revendication 1 ou 2 en tant que substance odorante et/ou aromatique.

4. Utilisation selon la revendication 3 pour conférer, modifier et/ou renforcer une note olfactive ou gustative fruitée (notamment abricot), fleurie (notamment lilas), sucrée et semblable à l'osmanthe.

5. Utilisation d'un composé selon la revendication 1 ou 2 pour rehausser l'odeur d'autres substances odorantes perçues par-dessus une solution aqueuse contenant un agent tensioactif.

6. Article comprenant un ou plusieurs composé/s de formule (A) selon la revendication 1 ou 2.

7. Article selon la revendication 6, comprenant ou formé de deux ou plusieurs composés de formule (A) selon la revendication 1 ou 2.

8. Article selon la revendication 6, comprenant ou formé d'un premier et d'un second composés de formule (A) selon la revendication 1, R étant identique dans le premier et dans le second composés, R¹ étant le méthyle et la double liaison associée du premier composé étant configurée (E) et celle du second composé étant configurée (Z).

9. Article selon l'une des revendications 6 à 8, comprenant une quantité totale de composés de formule (A) selon l'une des revendications 1 ou 2 qui suffit pour conférer, pour modifier et/ou pour renforcer une, deux, trois ou toutes les notes olfactives ou gustatives du groupe comprenant les notes fruitée (notamment abricot), fleurie (notamment lilas), sucrée et semblable à l'osmanthe.

10. Article selon l'une des revendications 6 à 9, l'article étant une composition de substances odorantes ou aromatiques, comprenant un ou plusieurs composé/s de formule (A) selon l'une des revendications 1 ou 2 ainsi qu'une ou plusieurs autre/s substance/s odorante/s resp. aromatique/s.

11. Article selon l'une des revendications 6 à 10, comprenant, en outre, un support ou un substrat qui est en contact direct avec le ou les composé/s de formule (A) resp. la composition de substances odorantes ou aromatiques.

12. Composition de substances odorantes ou aromatiques selon l'une des revendications 10 à 11, comprenant une quantité totale de composés de formule (A) selon la revendication 1 ou 2 représentant entre 0,001 et 70% en poids, de préférence entre 0,05 et 50% en poids et, plus préférablement encore, entre 0,5 et 25% en poids, de la quantité totale de la composition de substances odorantes resp. aromatiques.

13. Procédé pour conférer, modifier et/ou renforcer une odeur ou un goût, une quantité (a) d'un ou de plusieurs composé/s de formule (A) selon la revendication 1 ou 2 ou (b) d'une composition de substances odorantes ou aromatiques selon l'une des revendications 10 à 12 étant mise en contact ou mélangée avec un produit.

14. Procédé selon la revendication 13 pour conférer, modifier et/ou renforcer une odeur ou un goût comprenant une, deux, trois ou toutes les notes fruitée (notamment abricot), fleurie (notamment lilas), sucrée et semblable à l'osmanthe.

15. Procédé pour modifier et/ou renforcer une odeur ou un goût comprenant une, deux, trois ou toutes les notes fruitée (notamment abricot), fleurie (notamment lilas), sucrée et semblable à l'osmanthe, comprenant l'étape suivante:
- mélanger
une quantité d'un ou de plusieurs composé/s de formule (A) selon la revendication 1 ou 2
avec
une ou plusieurs autre/s substance/s odorante/s ou aromatique/s comprenant une, deux, trois ou toutes les notes fruitée (notamment abricot), fleurie (notamment lilas), sucrée et semblable à l'osmanthe,
la quantité utilisée de l'un ou des composé/s de formule (A) selon la revendication 1 ou 2 suffisant pour modifier et/ou renforcer sensoriellement l'impression olfactive resp. gustative des autres substances odorantes resp. aromatiques qui donnent naissance à une, deux, trois ou toutes les notes fruitée (notamment abricot), fleurie (notamment lilas), sucrée et semblable à l'osmanthe.

16. Procédé de fabrication d'un composé de formule (A) selon la revendication 1 ou 2, comprenant les étapes suivantes:
(i) préparation ou fabrication d'un composé de formule (C) 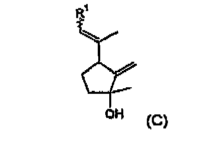 et
(ii) transformation du composé de formule (C) en une ou plusieurs étapes, de manière à ce qu'une transposition allylique et une éthérification aboutissent au composé de formule (A).

17. Procédé de fabrication d'un article selon l'une des revendications 6 à 12, comprenant l'étape suivante:
- mélange d'un ou de plusieurs composé/s de formule (A) selon la revendication 1 ou 2 avec d'autres composants, une quantité d'un ou de plusieurs composé/s de formule (A) suffisante pour conférer, pour modifier et/ou pour renforcer une note olfactive ou gustative dans l'article étant utilisée.
